# EUROPÄISCHE PATENTANMELDUNG

(11) **EP 4 487 898 A1**
(43) Veröffentlichungstag der Anmeldung: **08.01.2025**
(21) Anmeldenummer: 24184612.0
(22) Anmeldetag: 26.06.2024
(51) Int. Cl.: A61M 39/10, A61M 39/12, F16L 37/098, F16L 37/12, F16L 37/14

(54) **LÖSBARER KONNEKTOR MIT RASTELEMENT**

(30) Priorität: 05.07.2023 DE 102023117751
(71) Anmelder: B. Braun Avitum AG, 34212 Melsungen (DE)
(72) Erfinder: KNIERIM, Michael, 37242 Bad Sooden-Allendorf (DE)
(74) Vertreter: Winter, Brandl - Partnerschaft mbB

(57) **Zusammenfassung**

Die vorliegende Offenbarung betrifft einen medizinischen Konnektor (1) zum lösbaren Herstellen einer Fluidverbindung zwischen einer männlichen Konnektorkomponente (2) und einer weiblichen Konnektorkomponente (4) durch Einführen eines Einführabschnitts (6) der männlichen Konnektorkomponente in einen Aufnahmeabschnitt (8) der weiblichen Konnektorkomponente. Ein Rastelement (10) der weiblichen Konnektorkomponente ist beim Verbinden der beiden Konnektorkomponenten (2, 4) in eine Nut (12) an dem Einführabschnitt der männlichen Konnektorkomponente einrastbar ist und das Rastelement (10) ist zum Lösen der beiden Konnektorkomponenten (2, 4) senkrecht zu der Einführrichtung verschiebbar. Das Rastelement (10) ist beim Verschieben senkrecht der Einführrichtung durch einen Vorsprung (16) an der weiblichen Konnektorkomponente (4) deformierbar.

## Beschreibung

Die vorliegende Offenbarung betrifft einen, insbesondere medizinischen, lösbaren Konnektor/Konnektorsystem zum Herstellen einer Fluidverbindung mit einem Rastelement.

### Hintergrund der Offenbarung

Konnektoren der vorliegenden Gattung werden bei medizinischen Geräten zum Herstellen einer Fluidverbindung beispielsweise zwischen zwei hydraulisch oder pneumatisch zu koppelnden Bauteilen eingesetzt. Dabei können Fluidleitungen miteinander verbunden oder eine Fluidleitung an ein medizinisches Gerät angeschlossen werden. Für medizinische Anwendungen werden oft Einmalschläuche verwendet, die nach einmaligem Gebrauch entsorgt werden. Da Benutzer solche Einmalschläuche regelmäßig an- und abstecken, müssen die Konnektoren für die Einmalschläuche besonders gut handhabbar sein. D.h. eine Fluidleitung muss einfach und schnell an eine andere Fluidleitung oder das medizinische Gerät koppelbar sein. Gleichzeitig muss sichergestellt sein, dass die durch den entsprechenden Konnektor hergestellte Fluidverbindung fest und komplett geschlossen/dicht ist und keine Flüssigkeit aus der Verbindung austritt, da Leckagen bei medizinischen Anwendungen die Gesundheit eines Patienten gefährden können.

Medizinische Leitungs-/Schlauchverbindungen sind folglich in allgemeiner Definition Geräte/Bauteile, die verwendet werden, um medizinische Schläuche miteinander oder mit anderen medizinischen Geräten wie Pumpen, Filter, Ventilen und dergleichen Fluidelemente zu verbinden. Sie ermöglichen den Fluss von Flüssigkeiten, Gasen und anderen Medien durch den Schlauch.

Die Funktionsweise von medizinischen Leitungs-/Schlauchverbindungen hängt von ihrem Typ und Modell ab, aber im Allgemeinen verfügen sie über eine Kupplung, die auf einer Seite des Konnektors angeordnet/ausgebildet ist, und einen Adapter oder einen Hohlkörper auf der anderen Seite des Konnektors, wobei der Adapter/Hohlkörper den Schlauch aufnehmen kann. Die Verbindung wird hergestellt, indem der Schlauch in den Adapter eingesetzt wird und die Kupplung mit einer anderen Kupplung oder einem Adapter verbunden wird, die/der an der anderen Leitung oder am medizinischen Gerät angeordnet ist. Insoweit ähnelt das vorstehend beschriebene Konnektorprinzip allgemein einer handelsüblichen Schlauch-Anschlusskonstruktion, wie sie bei auch bei herkömmlichen Wasserschläuchen verwendet werden.

### Stand der Technik

Daher sind aus dem Stand der Technik auch eine große Anzahl an Konnektoren bekannt, die sowohl einfach handhabbar als auch sicher verbindbar sind.

Aus der DE 195 22 690 A1 ist beispielhaft eine Steckverbindung für den Anschluss von Rohr- und Schlauchleitungen bei Kraftfahrzeugen mit einer Rastfeder bekannt. Dabei rastet die Rastfeder eines Verbindungsteils in einer Nut an einem anderen Verbindungsteil ein und fixiert die beiden Verbindungsteile somit aneinander. Die Verbindung kann wieder gelöst werden, indem die Rastfeder von Hand in eine Rastnocke geschoben wird. Dadurch wird die Rastfeder deformiert und gibt die Verbindung frei. Das händische Verbiegen der Rastfeder benötigt aber einen erheblichen Kraftaufwand eines Benutzers.

Die DE 37 29 570 A1 zeigt ebenfalls einen Verbinder zum Verbinden von Schläuchen. Der Verbinder weist ein männliches Verbindungsteil mit einer Nut auf, in die ein Rastelement eines weiblichen Verbindungsteils einrastet, um eine sichere Verbindung des männlichen mit dem weiblichen Verbindungsteil zu gewährleisten. Zum Lösen der Konnektorverbindung kann das Rastelement von den beiden Verbindungsteilen wegzogen werden. Dabei wird das Rastelement von dem männlichen Verbindungsteil aufgebogen. Das Aufbiegen an dem männlichen Verbindungsteil kann aber dazu führen, dass das Rastelement nicht weit genug deformiert wird und in der Nut hängen bleibt. Dadurch kann die Verbindung nicht oder nur schwer gelöst werden.

Ferner sind auf dem Markt verschiedene Lösungen von verschiedenen Konnektoren bzw. (Fluid-)Kupplungen verfügbar. Beispielsweise ist es bekannt, Fluidkupplungen mit einer (universalen) Mutter zu befestigen. In diesem Fall kann aber kein Schlauch mit Bezug auf einen bestimmten Konnektor formatiert werden, was für den Montageablauf ungünstig ist. Bei standardmäßigen Kollektoren ist es ferner schwierig, den Innendurchmesser des Konnektors mit den Durchmessern der verwendeten Fluidleitungen oder Silikonschläuche zusammenzufügen. Ferner ist es kompliziert, einen gekauften Konnektor oder eine gekaufte Konnektorkomponente mit einem selbst angefertigten Gegenstück zu kombinieren.

### Zusammenfassung der Offenbarung

Somit ist es die Aufgabe der vorliegenden Offenbarung, die Nachteile des Standes der Technik zu überwinden oder zumindest zu vermindern und insbesondere einen medizinischen Konnektor bereitzustellen, der einfach und kostengünstig gefertigt werden kann, sicher verschließbar, einfach handhabbar und insbesondere einfach lösbar ist.

Diese Aufgabe wird offenbarungsgemäß durch einen Konnektor mit den Merkmalen des Anspruchs 1 gelöst. Vorteilhafte Weiterbildungen der vorliegenden Offenbarung sind Gegenstand der beigefügten Unteransprüche.

Die vorliegende Offenbarung betrifft demzufolge einen (medizinischen) Konnektor bzw. Konnektoraufbau / Konnektorsystem zum lösbaren Herstellen einer Fluidverbindung zwischen einer männlichen Konnektorkomponente und einer weiblichen Konnektorkomponente des Konnektorsystems durch Einführen zumindest eines Einführabschnitts der männlichen Komponente in einen Aufnahmeabschnitt der weiblichen Konnektorkomponente. Ein (elastisches/flexibles) (separates) Rastelement der weiblichen bzw. auf Seiten der weiblichen Konnektorkomponente ist beim (zum) Verbinden der beiden Konnektorkomponenten in eine Nut/einen Hinterschnitt (vorzugsweise gebildet durch einen Rastvorsprung und/oder Rastrücksprung) an dem Einführabschnitt der männlichen Konnektorkomponente (automatisch/selbsttätig weil elastisch/flexibel) einrastbar und das Rastelement ist zum (für ein) Lösen der beiden Konnektorkomponenten (voneinander) in eine Richtung (im Wesentlichen) senkrecht zur Einführrichtung (manuell) verschieb- oder bewegbar. Offenbarungsgemäß ist das Rastelement beim Verschieben/Bewegen (im Wesentlichen) in Richtung hin zu einer Mittelachse der beiden Konnektorkomponenten nach radial innen quer zur Einführrichtung durch einen Vorsprung an der weiblichen Konnektorkomponente deformierbar, derart, dass dadurch der Rasteingriff gelöst/freigegeben wird. Konkreter kann das Rastelement in Richtung der Mittelachse der beiden Konnektorkomponenten nach radial innen gedrückt / geschoben werden.

In anderen Worten wird zur Herstellung einer Fluidverbindung der männliche Einführabschnitt eines ersten Konnektorteils zunächst in den weiblichen Aufnahmeabschnitt eines zweiten Konnektorteils eingeführt oder eingesteckt. Somit wird eine (dichte) Fluidverbindung zwischen jeweiligen (zentralen oder mittigen) Fluidkanälen der Konnektorteile hergestellt. Beim Einführen / Einstecken des männlichen Einführabschnitts in den weiblichen Aufnahmeabschnitt wird das am weiblichen Aufnahmeabschnitt angeordnete / gelagerte Rastelement vom männlichen Einführabschnitt derart elastisch verformt (aufgeweitet, aufgespreizt, verbogen), dass das Rastelement anschießend (d.h. mit Erreichen einer Kopplungsendposition der beiden Konnektorteile) selbsstätig in eine Nut am Einführabschnitt des männlichen Konnektorteils einrastet bzw. zurückfedert. Somit werden die beiden Konnektorteile durch die Kombination aus dem (separaten) Rastelement, gelagert am weiblichen Kupplungsteil und der Nut/Rücksprung/Vorsprung, ausgebildet am männlichen Kupplungsteil in der Kopplungsendposition (quasi unlösbar) zusammengehalten. Eine feste (im Sinne eines unbeabsichtigten Lösens) Konnektorverbindung ist dadurch herstellbar bzw. hergestellt. Zum (beabsichtigten) Lösen der beiden Konnektorteile voneinander ist das Rastelement durch eine externe Kraft derart (quer zur Einsteckrichtung) relativ zum weiblichen Konnektorteil verschiebbar oder bewegbar, sodass das Rastelement durch die Verschiebung/Bewegung die Nut freigibt bzw. aus der Nut bewegt wird, wodurch die beiden Konnektorteile relativ zueinander entgegen der Einsteckrichtung bewegbar sind. Dabei kann das Rastelement vorzugsweise zur Mitte der beiden Konnektorteile hin (also nach radial innen) gedrückt werden. Beim Bewegen des Rastelements, um die Konnektorverbindung zu lösen, wird das Rastelement bevorzugt an einer (quer zur Einsteckrichtung sich erhebenden) Rampe oder Abgleitfläche/-kante am weiblichen Konnektorteil (elastisch) deformiert bzw. aufgebogen (im Sinn von in Querrichtung zur Einsteckrichtung verbogen), wodurch das Rastelement nicht mehr in der Nut aufgenommen oder fixiert ist, bzw. aus der Nut herausgenommen wird. Dadurch sind der weibliche und der männliche Konnektorteil (wieder) relativ zueinander bewegbar.

Konkret liegt der Kern der Offenbarung also darin, dass das Rastelement zum Lösen der Konnektorverbindung (quer zur Einsteckrichtung) manuell/aktiv verschiebbar / bewegbar ist bzw. verschoben/bewegt werden muss, wobei die Querverschiebung des Rastelements (mit vergleichsweise geringem Kraftaufwand) zu einer (vergleichsweise kraftaufwändigen) elastischen Verformung/Aufspreizen vorzugsweise durch die Rampe am weiblichen Konnektorteil übersetzt wird, wohingegen zum Verrasten der beiden Konnektorteile in der Kopplungsendposition das Rastelement selbsttätig/automatisch beim Einstecken des männlichen Konnektorteils in das weibliche Konnektorteil (durch den Vorsprung am männlichen Konnektorteil) zunächst quer zur Steckrichtung deformierbar ist bzw. deformiert wird und sich anschließend durch Zurückfedern in seine Rastposition bewegt/federentspannt.

Der offenbarungsgemäße Konnektor weist dabei die folgenden Vorteile auf:
Durch die Kombination aus dem Rastelement und dem Hinterschnitt/der Nut kann eine in Steckrichtung feste Konnektorverbindung entstehen. Ein Auslaufen von Fluiden, die durch den Konnektor fließen, kann durch die feste Verbindung verhindert werden. Durch die Elastizität des Rastelements muss die Konnektorverbindung nicht händisch angeschlossen oder verrastet werden, sondern erfolgt quasi automatisch mit dem Einsteckvorgang. D.h. es wird verhindert, dass ein Benutzer das axiale Fixieren der beiden Konnektorkomponenten vergisst, da die beiden Konnektorkomponenten "automatisch" bzw. von selber fixiert werden. Ferner sind die beiden Konnektorkomponenten durch das Verschieben des Rastelements einfach und intuitiv voneinander lösbar. Insbesondere kann die Verbindung mit einer Hand gelöst werden. Zum Lösen der beiden Konnektorkomponenten voneinander muss nicht viel Kraft von dem Benutzer aufgebracht werden. Besonders bevorzugt kann das Rastelement mit einem Handballen des Benutzers nach radial innen gedrückt / geschoben werden.

Vorzugsweise weist die männliche Konnektorkomponente die Rampe/einen Vorsprung auf, die nach radial außen aus dem Einführabschnitt der männlichen Konnektorkomponente vorsteht. Das Rastelement kann beim Einführen der männlichen Konnektorkomponente durch die Rampe elastisch verformt oder aufgebogen werden. Die Rampe biegt also das Rastelement beim Verbinden der beiden Konnektorkomponenten auf und ist derart gestaltet, dass das Rastelement nach Passieren der Rampe an einer steilen Flanke der Rampe in die Nut einrastet. Somit kann die Konnektorverbindung durch das Rastelement fest verbunden und gesichert werden. Alternativ hierzu wäre es aber auch denkbar, eine Abgleitkante am weiblichen Konnektorteil vorzusehen und die Rampe am Rastelement auszubilden, wodurch ebenfalls die vorstehend beschriebene elastische Biegebewegung des Rastelements bei dessen Querverschiebung erreichbar ist.

Besonders bevorzugt wird das Rastelement zum Lösen der beiden Konnektorkomponenten in Richtung der beiden Konnektorkomponenten bzw. hin zum zentralen Fluidkanal durch den Konnektor nach radial innen verschoben. D.h. der Benutzer kann derart auf das Rastelement drücken, dass sich das Rastelement zum zentralen Fluidkanal hin verschiebt. Der Benutzer kann das Rastelement beispielsweise mit einem Handballen/Finger nach radial innen drücken. Somit kann der Benutzer das Rastelement ohne großen Kraftaufwand und mit ergonomischer (Hand-)Haltung betätigen. Dadurch kann insbesondere ein (Ein-)Drückmechanismus oder eine Drückfunktion des Rastelements bereitgestellt werden.

Alternativ ist es aber grundsätzlich auch möglich, durch radiales Herausziehen des Rastelements ein Verbiegen des Rastelements ein außer Rastengriff kommen zu bewirken. So kann das Rastelement vorzugsweise zum Lösen der beiden Konnektorkomponenten (zusätzlich) nach radial außen verschiebbar, insbesondere ziehbar, sein. Somit kann insbesondere eine kombinierte Zieh- und Drückfunktion des Rastelements bereitgestellt werden.

Nach einem weiteren optionalen Aspekt der vorliegenden Offenbarung kann der Vorsprung als eine Rampe an der weiblichen Konnektorkomponente ausgebildet sein. Das Rastelement bzw. ein Abschnitt des Rastelements kann an der Rampe abgleiten, wobei das Rastelement durch das Abgleiten entlang der Rampe aufbiegbar ist. Die Rampe ist insbesondere derart ausgerichtet, dass eine (radial nach außen) ansteigende Flanke der Rampe zu dem Rastelement hin ausgerichtet ist. Bei Betätigung/Verschieben des Rastelements (durch den Benutzer) kann das Rastelement die Flanke hinaufgleiten und wird somit durch die Rampe elastisch aufgebogen. Dadurch gibt das aufgebogene Rastelement die männliche Konnektorkomponente und, insbesondere den Hinterschnitt der männlichen Konnektorkomponente, frei. Sollte das Rastelement in zwei Richtungen (nach radial innen und außen) quer zur Einführrichtung verschiebbar sein, kann die weibliche Konnektorkomponente auch zwei Vorsprünge bzw. Rampen aufweisen, um die kombinierte Zieh- und Drückfunktion zu verwirklichen.

Nach einem optionalen Aspekt der vorliegenden Offenbarung weist die weibliche Konnektorkomponente in Verschiebungsrichtung des Rastelements hinter dem Vorsprung eine (Ein-)Rastnut auf, in die das Rastelement nach dem Deformieren oder Aufbiegen durch den Vorsprung einrastet. Die Einrastnut kann dabei in Bewegungsrichtung des Rastelements derart zu dem Vorsprung platziert sein, dass das Rastelement zuerst deformiert und insbesondere aufgebogen wird und anschließend zurückschnappt. Das Rastelement kann also zum Lösen der Konnektorverbindung erst elastisch deformiert werden, um die männliche Konnektorkomponente freizugeben und kann anschließend in die Einrastnut einschnappen oder einrasten, damit die Verbindung offen bzw. unverrastet bleibt, d.h. die beiden Konnektorkomponenten relativ zueinander beweglich bleiben. D.h. das Rastelement kann in der Einrastnut verbleiben und die Konnektorkomponenten können beispielsweise getrennt werden. Das Rastelement kann dabei also in einem gelösten bzw. offenen Zustand sein/verharren, der auch als geöffnete Kupplung bezeichnet werden kann. Die Einrastnut kann bei einer Verschiebungsrichtung nach radial innen und/oder bei der Verschiebungsrichtung nach radial außen jeweils in Verschiebungsrichtung hinter dem Vorsprung angeordnet sein. D.h. die weibliche Konnektorkomponente kann auch zwei Einrastnuten aufweisen, wobei eine in jeder Verschiebungsrichtung (hinter dem Vorsprung) angeordnet ist.

Vorzugsweise ist das Rastelement zum Lösen der beiden Konnektorkomponenten nach radial innen hin zu den beiden Konnektorkomponenten verschiebbar und für ein Blockieren/Verrasten nach radial außen verschiebbar, insbesondere ziehbar. Hierfür hat oder bildet das Rastelement eine Art Öse oder Bügel, worin der Benutzer beispielsweise einen oder mehrere Finger in das Rastelement einhaken kann, um das Rastelement in Richtung hin zum Benutzer zu ziehen oder weg von dem Benutzer zu drücken.

Nach einem weiteren optionalen Aspekt der vorliegenden Offenbarung ist das Rastelement beim Drücken nach radial innen (oder alternativ beim Ziehen nach radial außen) durch den Vorsprung an der weiblichen Konnektorkomponente deformierbar und insbesondere aufbiegbar. Auch bei der manuellen Betätigung des Rastelements in die Freigaberichtung wird das Rastelement von dem Vorsprung bzw. der Rampe auf Seiten der weiblichen Konnektorkomponente auf- oder umgebogen und kann anschließend in die Einrast- oder Haltenut hinter dem Vorsprung/Rampe der weiblichen Konnektorkomponente einrasten, um die die Konnektorverbindung freigebende Position beizubehalten. Wenn das Rastelement durch dessen Verschieben eine bestimmte Position relativ zur weiblichen Konnektorkomponente einnimmt, beispielsweise nach radial außen aus dem Konnektor vorsteht (oder alternativ in diesem eingetaucht ist), kann der Benutzer deutlich erkennen, dass das Rastelement im gelösten/freigebendem Zustand ist.

Vorzugsweise kann die Einrast- oder Haltenut in einer Symmetrieachse der weiblichen Konnektorkomponente angeordnet sein. Die Symmetrieachse der weiblichen Konnektorkomponente erstreckt sich parallel oder entlang des zentralen Fluidkanals in Längsrichtung der weiblichen Konnektorkomponente. D.h. die Einrastnut ist im Querschnitt durch die weibliche Konnektorkomponente gesehen mittig angeordnet. Somit kann das Rastelement auch beim Ziehen in Richtung hin zum Benutzer in die Einrastnut einrasten. Wenn der Benutzer das Rastelement zu sich hinzieht, quert das Rastelement von seiner (vorgespannten) Ausgangsposition aus, die auf der Seite der weiblichen Konnektorkomponente liegt, die dem Benutzer abgewandt ist, den zentralen Fluidkanal. Bei der Ziehbewegung wird das Rastelement erst durch den Vorsprung an der weiblichen Konnektorkomponente (radial nach Außen) deformiert oder aufgebogen und rastet anschließend in die Einrastnut an der weiblichen Konnektorkomponente ein, um in dieser aufgebogenen sowie ausgezogenen Position gehalten zu werden.

Das Rastelement kann beispielsweise als ein vorzugsweise U-förmiger Bügel ausgeführt sein. Der Bügel kann dabei einen Greifabschnitt (Querbalken), der zum Greifen vom Benutzer geeignet ist und einen Deformationsabschnitt (zwei im Wesentlichen parallele Schenkel) aufweisen. Der Deformationsabschnitt ist dazu geeignet, von dem Vorsprung aufgebogen zu werden. Da der Bügel beispielsweise aus einem dünnen Federdraht oder einem entsprechenden anderen Metall besteht, kann der Bügel auch durch geringen Kraftaufwand elastisch deformiert werden. Der Deformationsabschnitt kann in die Nut an der männlichen Komponente einrasten und zum Lösen der Verbindung aufgebogen werden. Um letzteres noch weiter zu vereinfachen, können die beiden Schenkel des U-förmigen Bügels auch so gestaltet sein, dass sie in deren Längserstreckung unterschiedliche Abstände aufweisen. D.h. es ist denkbar, den Schenkelabstand im Bereich ihrer freien Endabschnitte größer zu machen als im Bereich ihrer Querbalken-nahen Endabschnitte. Wird in diesem Fall der Bügel in Querrichtung zurückgedrückt, muss dieser nichtmehr soweit aufgebogen werden, um dann die männliche Konnektorkomponente im Bereich der freien Endabschnitte axial freizugeben.

Vorzugsweise kann der Konnektor und insbesondere die weibliche Konnektorkomponente, eine Montageschräge zum Montieren des in diesem Fall als U-förmiger Bügel ausgebildeten Rastelements aufweisen. Die Montageschräge kann als eine Phase oder Schräge am Gehäuse der weiblichen Komponente ausgebildet sein und die Montage des Rastelements vereinfachen. Beim Montieren des Rastelements kann das Rastelement manuell aufgebogen werden und in eine Gleitnut an der weiblichen Komponente eingesetzt werden. Durch die Montageschräge kann es einfacher werden, das Rastelement in die Gleitnut einzusetzen.

Nach einem weiteren optionalen Aspekt der vorliegenden Offenbarung kann die Rampe/der Vorsprung an dem Einführabschnitt der männlichen Konnektorkomponente eine bezüglich der Längsachse des Einführabschnitts flache Flanke und eine steile Flanke aufweisen. Die flache Flanke kann dabei insbesondere einem distalen Endabschnitt des Einführabschnitts zugewandt sein, der in Richtung zur weiblichen Konnektorkomponente zeigt. Dabei kann der distale Endabschnitt die Seite des Konnektors sein, die beim Verbinden in Richtung zur weiblichen Konnektorkomponente zeigt bzw. beim Einführen des Einführabschnitts zuerst in den Aufnahmeabschnitt eintaucht. D.h. die flache Flanke zeigt beim Einführen nach vorne in Bewegungsrichtung. Durch die Interaktion mit der flachen Flanke wird das Rastelement langsam aufgebogen. Die Kombination aus der steilen Flanke und der Nut kann den Hinterschnitt an der männlichen Konnektorkomponente bilden. Insbesondere kann die steile Flanke die effektive Tiefe der Nut vergrößern, sodass das Rastelement in der Nut sicher verrasten kann. Somit kann eine sichere Konnektorverbindung gewährleistet werden und das Risiko eines ungewollten Lösens minimiert werden.

Die weibliche Konnektorkomponente weist ferner zumindest zwei Schnapphaken auf. Durch die Schnapphaken kann die weibliche Konnektorkomponente bzw. der gesamte Konnektor mit einem Aufnahmeblech verrrastet werden. Das Aufnahmeblech hat eine längliche Aufnahmenut, in die die weibliche Konnektorkomponente bzw. der gesamte Konnektor eingesetzt werden kann. Die Schnapphaken können mit einer entsprechenden (Verrastungs-)Kontur an dem Aufnahmeblech verrrasten. Somit können mehrere Konnektoren neben- oder übereinander in ein Aufnahmeblech eingesetzt und darin fixiert werden. Das Aufnahmeblech kann beispielsweise die Front eines medizinischen Geräts ausbilden.

Kurzbeschreibung der Figuren
Fig. 1 zeigt eine perspektivische Ansicht eines Konnektors gemäß einer ersten Ausführungsform der vorliegenden Offenbarung;
Fig. 2 zeigt eine perspektivische Ansicht eines Konnektors gemäß der ersten Ausführungsform der vorliegenden Offenbarung mit getrennten Konnektorkomponenten;
Fig. 3 zeigt einen Längsschnitt durch den Konnektor gemäß der ersten Ausführungsform der vorliegenden Offenbarung;
Fig. 4 zeigt einen Querschnitt durch den Konnektor gemäß einer zweiten Ausführungsform der vorliegenden Offenbarung;
Fig. 5 zeigt eine perspektivische Ansicht des Konnektors gemäß der zweiten Ausführungsform der vorliegenden Offenbarung;
Fig. 6 zeigt eine perspektivische Ansicht einer männlichen Konnektorkomponente des Konnektors gemäß der ersten sowie zweiten Ausführungsform der vorliegenden Offenbarung;
Fig. 7 zeigt eine perspektivische Ansicht einer weiblichen Konnektorkomponente des Konnektors gemäß der zweiten Ausführungsform der vorliegenden Offenbarung;
Fig. 8 zeigt eine perspektivische Ansicht eines Konnektors gemäß einer dritten Ausführungsform der vorliegenden Offenbarung; und
Fig. 9 zeigt eine perspektivische Ansicht eines Konnektors gemäß einer vierten Ausführungsform der vorliegenden Offenbarung; und
Fig. 10 zeigt eine perspektivische Ansicht eines Konnektors gemäß einer fünften Ausführungsform der vorliegenden Offenbarung.

### Detaillierte Beschreibung der Figuren

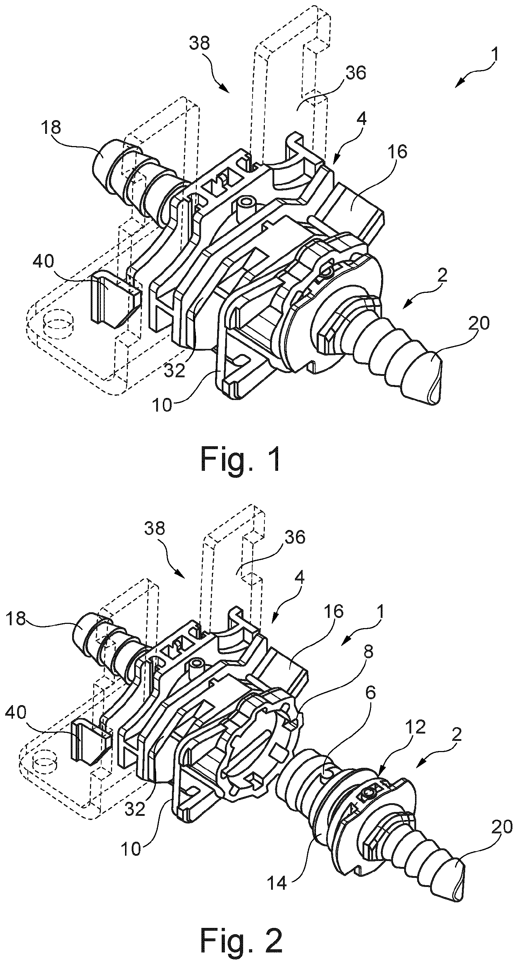

Figuren 1 und 2 zeigen einen medizinischen Konnektor/Konnektorsystem 1 zum Herstellen einer lösbaren Fluidverbindung zwischen einer männlichen Konnektorkomponente 2 als ein erster Bestandteil des Konnektors/Konnektorsystems und einer weiblichen Konnektorkomponente 4 als ein zweiter Bestandteil des Konnektors/Konnektorsystems. Dabei wird ein Einführabschnitt 6 der männlichen Konnektorkomponente 2 in einen Aufnahmeabschnitt 8 der weiblichen Konnektorkomponente 4 bzw. in einen Aufnahmeraum des Aufnahmeabschnitts 8 eingeführt. Die weibliche Konnektorkomponente 4 weist ein Rastelement bzw. einen (U-förmigen) Verschlussbügel 10 auf, der in eine Nut 12 an dem Einführabschnitt 6 der männlichen Konnektorkomponente 2 einrastbar ist. Der Verschlussbügel 10 ist zum Lösen der beiden Konnektorkomponenten 2 und 4 in eine Richtung quer, vorzugsweise senkrecht zur Einführrichtung verschiebbar an der weiblichen Konnektorkomponente 4 gelagert/gehalten.

Fig. 2 zeigt den Konnektor 1 mit separaten oder voneinander getrennten Konnektorkomponenten 2, 4. Der Einführabschnitt 6 ist dafür ausgerichtet, in den Aufnahmeabschnitt 8 der weiblichen Konnektorkomponente 4 eingeführt zu werden. Der Einführabschnitt 6 weist eine axial sich erstreckende Rampe 14 auf, die radial aus dem Einführabschnitt 6 hervorsteht. Die weibliche Konnektorkomponente 4 weist den Aufnahmeabschnitt 8 auf, in den der Einführabschnitt 6 der männlichen Konnektorkomponente 2 eingesteckt wird. Die weibliche Konnektorkomponente 4 trägt verschiebbar den Verschlussbügel 10 als separater Bestandteil der weiblichen Konnektorkomponente 4, der derart elastisch verform- oder deformierbar ist (d.h. die frei auskragenden Schenkel des U-förmigen Bügels sind elastisch aufspreizbar, dass er beim Einführen des Einführabschnitts 6 in den Aufnahmeabschnitt 8 von der Rampe 14 auseinandergebogen wird und nach dem Passieren der Rampe 14 in die Nut 12 einrastet. Somit können die beiden Konnektorkomponenten 2, 4 fest miteinander verbunden und verrastet werden. Die Kombination der Rampe 14 und der benachbarten (axial sich unmittelbar anschließenden) Nut 12 bildet einen Hinterschnitt an der weiblichen Konnektorkomponente 4, die in Axialrichtung/entgegen der Einsteckrichtung wirkt.

D. h. während die beiden Konnektorkomponenten 2, 4 verbunden werden, ändert sich der Verfahrweg des Einführabschnitts 6 relativ zum Aufnahmeabschnitt 8. Somit wird der Verschlussbügel 10 (dessen Schenkel) beim Einschieben des Einführabschnitts 6 zuerst durch die Rampe 14 auseinandergebogen. Wenn die Rampe 14 den Verschlussbügel 10 im Laufe des Verfahrwegs des Einführabschnitts 6 passiert hat, federt der elastische Verschlussbügel 10 hinter der Rampe 14 radial nach innen zurück. D.h. der elastische Verschlussbügel 10, der von der Rampe 14 (elastisch) auseinander- oder aufgebogen wurde, kann sich wieder entspannen und in seine Ausgangsposition zurückkehren. D.h. der Verschlussbügel 10 schnellt nach radial innen in die Nut 12 zurück. Dabei rastet der Verschlussbügel 10 in die Nut 12 ein, die direkt hinter (in Einsteckrichtung gesehen) der Rampe 14 angeordnet ist. Somit kann eine sichere Fixierung der beiden Konnektorkomponenten 2, 4 durch den Verschlussbügel 10 gewährleistet werden. Beim Verbinden der beiden Konnektorkomponenten 2, 4 ist der Verschlussbügel 10 durch die elastische Verformung vorgespannt. Dadurch kehrt der Verschlussbügel 10 immer in seine (nicht verformte) Ausgangsposition zurück, was verhindert, dass ein Benutzer das Verrasten der beiden Konnektorkomponenten 2, 4 vergessen kann. Somit bewirkt der Verschlussbügel 10 eine automatische Verriegelung.

Zum Lösen der beiden Konnektorkomponenten 2, 4 kann der Verschlussbügel 10 in eine Richtung quer (vorzugsweise senkrecht) zur Einführrichtung des Einführabschnitts 6 verschoben werden. Die weibliche Konnektorkomponente 4 hat einen Vorsprung 16 mit einer Flanke, die zum Verschlussbügel 10 hin ausgerichtet ist. Wird der Verschlussbügel 10 betätigt und dabei senkrecht zur Einführrichtung in Richtung der beiden Konnektorkomponenten 2, 4 geschoben, gleitet der Verschlussbügel 10 zumindest abschnittsweise die Flanke des Vorsprungs 16 hinauf (d.h. radial nach außen). Dadurch wird der Verschlussbügel 10 zumindest abschnittsweise elastisch deformiert bzw. auseinandergebogen. Durch die Deformation greift der Verschlussbügel 10 nicht mehr in die Nut 12 ein. Die Rampe 14 kann den Verschlussbügel 10 passieren und die männliche Konnektorkomponente 2 kann relativ zur weiblichen Konnektorkomponente 4 bewegt werden. Somit können die beiden Konnektorkomponenten 2, 4 voneinander getrennt werden.

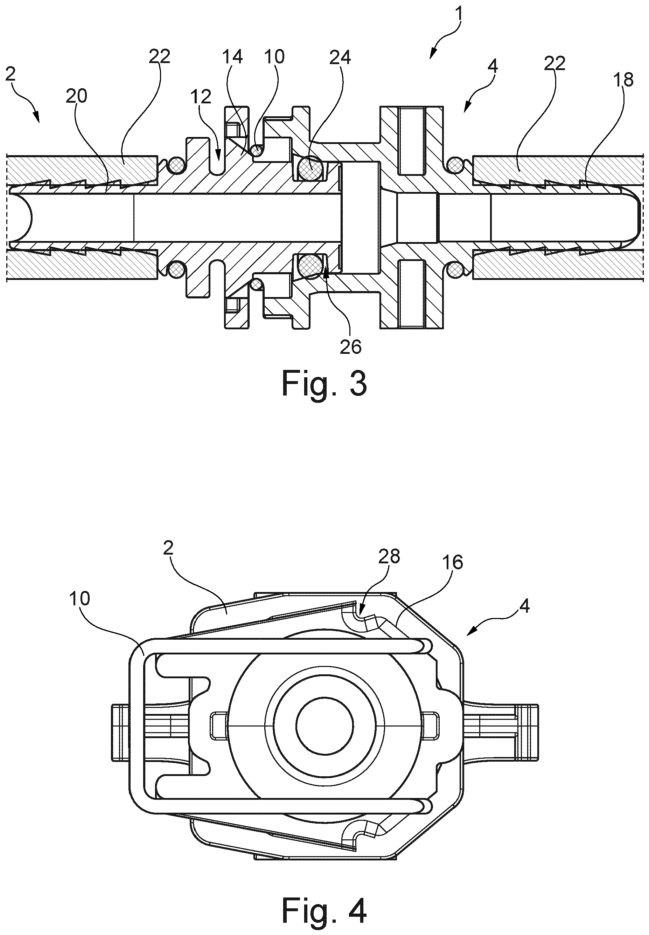

Dem Aufnahmeabschnitt 8 liegt ein (Anschluss-)Stutzen (Schlauchnippel) 18 gegenüber, der dafür ausgebildet ist, mit einer zweiten Fluidleitung (in Fig. 3 dargestellt) verbunden zu werden. Dem Einführabschnitt 6 liegt ein weiterer (Anschluss-)Stutzen (Schlauchnippel) 20 gegenüber, der dafür vorbereitet ist mit einer ersten Fluidleitung bzw. einem Schlauch 22 (in Fig. 3 dargestellt), insbesondere einem Silikonschlauch, verbunden zu werden. Der Einführabschnitt 6 weist ferner eine Dichtung 24 auf, die in einer Dichtungsnut 26 an einem distalen Endabschnitt des Einführabschnitts 6 sitzt. Die weibliche Konnektorkomponente 4 weist eine Montageschräge 32 für den Verschlussbügel 10 auf. Dadurch kann der Verschlussbügel 10 leichter montiert werden.

Die weibliche Konnektorkomponente 4 weist ferner zumindest zwei Schnapphaken 40 auf. Durch die Schnapphaken 40 kann die weibliche Konnektorkomponente 4 bzw. der gesamte Konnektor 1 mit einem Aufnahmeblech 36 verrrastet werden. Das Aufnahmeblech 36 hat eine längliche Aufnahmenut 38, in die die weibliche Konnektorkomponente 4 bzw. der gesamte Konnektor 1 eingesetzt werden kann. Die Schnapphaken 40 können mit einer entsprechenden (Verrastungs-)Kontur an dem Aufnahmeblech 36 verrrasten. Somit können mehrere Konnektoren 1 neben- oder übereinander in ein Aufnahmeblech 36 eingesetzt und darin fixiert werden. Das Aufnahmeblech 36 kann beispielsweise die Front eines medizinischen Geräts (nicht dargestellt) ausbilden. Die Schnapphaken 40 sind vorzugsweise jeweils seitlich an der weiblichen Konnektorkomponente 4 angeordnet und stehen von dem Grundkörper der weiblichen Konnektorkomponente 4 radial nach außen vor. Die Schnapphaken 40 sind dabei diametral gegenüber angeordnet.

Fig. 3 zeigt einen Längsschnitt durch den verbundenen Konnektor 1. Dabei ist gezeigt, wie der Verschlussbügel 10 in der Nut 12 anliegt, um die beiden Konnektorkomponenten 2, 4 aneinander zu befestigen. Die Nut 12 schließt an eine steile Flanke der Rampe 14 an. D.h. die steile Flanke und die Nut 12 ergeben zusammen eine Wand oder einen Hinterschnitt, dessen effektive Höhe so hoch ist, dass der Verschlussbügel 10 nicht über den Hinterschnitt rutschen kann. Somit ist der Verschlussbügel 10 fest in der Nut 12 aufgenommen. Die Dichtung 24 ist in der Dichtungsnut 26 des Einführabschnitts 6 aufgenommen.

Die Figur 3 zeigt den Konnektor 1 während des Schließvorgangs. Der Verschlussbügel liegt an der Schräge an, welche den Verschlussbügel beim Schließvorgang aufweitet und anschließend in den Hinterschnitt springen lässt (geschlossener Zustand).

Fig. 4 zeigt einen Querschnitt durch den Konnektor 1 gemäß einer zweiten Ausführungsform. Dabei wird der Verschlussbügel 10 nicht in Richtung der beiden Konnektorkomponenten 2, 4 geschoben, sondern in die entgegengesetzte Richtung von den Konnektorkomponenten 2, 4 wegezogen. Der Verschlussbügel 10 gleitet den Vorsprung 16 hinauf und wird von dem Vorsprung 16 auseinandergebogen. Der Vorsprung 16 ist in der Richtung angeordnet, in die der Verschlussbügel 10 zum Lösen der Konnektorverbindung gezogen wird. Dadurch ist der Vorsprung 16 näher an einer zentralen Symmetrieachse des Konnektors 1 angeordnet als der Verschlussbügel 10. In Zugrichtung hinter dem Vorsprung 16 ist eine Einrastnut 28 angeordnet, in die der Verschlussbügel 10 im geöffneten Zustand einrasten kann. Somit wird der Verschlussbügel 10 im geöffneten Zustand gegen Einrasten gesichert. Die Konnektorverbindung ist somit offen und der Nutzer kann die beiden Konnektorkomponenten 2, 4 einfach trennen und anschließend wieder verbinden. Der Nutzer muss den Verschlussbügel 10 nicht im deformierten Zustand halten, was Kraft spart.

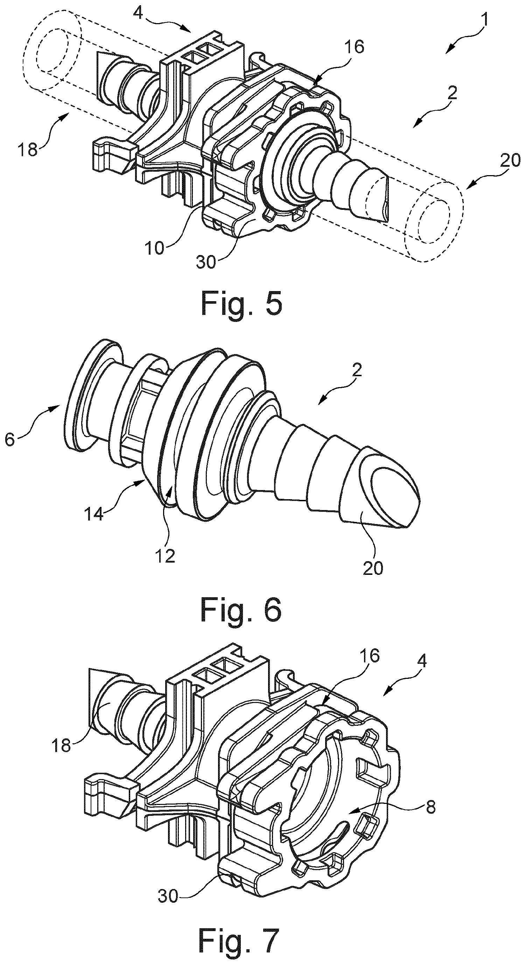

Fig. 5 zeigt den Konnektor 1 gemäß der zweiten Ausführungsform. Der Verschlussbügel 10 ist in einer verriegelten Stellung dargestellt. Damit sichert der Verschlussbügel 10 die Konnektorverbindung der beiden Konnektorkomponenten 2, 4 gegen ein ungewolltes Lösen. Zum Lösen der Konnektorverbindung wird der Verschlussbügel 10 von den beiden Konnektorkomponenten 2, 4 weggezogen und dabei vom Vorsprung 16 aufgebogen. Die weibliche Konnektorkomponente 4 weist einen Hinterschnitt 30 auf, der den Verschlussbügel 10 axial gegen Verschieben sichert.

Fig. 6 zeigt die männliche Konnektorkomponente 2. Die Rampe 14 der männlichen Konnektorkomponente 2 hat eine flache und eine steile Flanke. Die flache (Rampen-) Flanke zeigt in Richtung eines distalen Endabschnitts des Einführabschnitts 6. Der distale Endabschnitt ist der Endabschnitt, der beim Verbinden der Konnektorkomponenten 2, 4 in Richtung der weiblichen Konnektorkomponente 4, also in Einführrichtung, zeigt. D.h. der distale Endabschnitt wird beim Verbinden zuerst in den Aufnahmeabschnitt 8 eingeführt. Somit wird die flache Flanke zuerst in den Aufnahmeabschnitt 8 eingeführt. Die Nut 12 an dem Einführabschnitt 6 ist in Einführrichtung direkt hinter der Rampe 14 angeordnet.

Fig. 7 zeigt die weibliche Konnektorkomponente 4 ohne den Verschlussbügel 10. Der Verschlussbügel ist in dem Hinterschnitt 30 aufgenommen und somit axial gesichert. Die Form der weiblichen Konnektorkomponente 4 bildet den Hinterschnitt 30 derart aus, das der Verschlussbügel 10 darin geführt ist. Der Verschlussbügel 10 ist lediglich in die Richtung senkrecht zur Einführrichtung beweglich.

Der Konnektor 1 gemäß der ersten und zweiten Ausführungsform ist vorzugsweise durch Spritzguss gefertigt. Fertigungsbedingt weist der Konnektor 1, wie in den Figuren 1 bis 7 gezeigt, an seiner Außenoberfläche eine Anzahl an Verstärkungsrippen und Phasen auf.

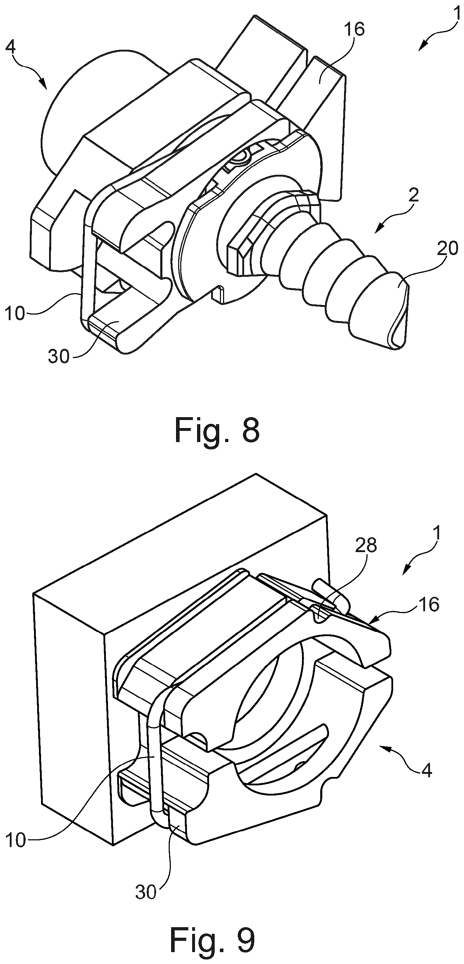

Fig. 8 zeigt den Konnektor 1 gemäß einer dritten Ausführungsform. Der Konnektor 1 ist im Gegensatz zur ersten und zweiten Ausführungsform (aus einem Block) gefräst bzw. anderweitig spanend aus einem Stück gefertigt. Daher ist die Oberfläche des Konnektors 1 ohne Verstärkungsrippen ausgeführt. Der Konnektor 1 gemäß der dritten Ausführungsform weist ebenfalls den Verschlussbügel 10 und den Vorsprung 16 auf. Dabei ist der Verschlussbügel 10 lediglich nach innen verschieb- oder drückbar, sodass der Verschlussbügel 10 den Vorsprung 10 Fertigung durch ein spanendes Fertigungsverfahren kann insbesondere bei Kleinserien und bei besonders komplexen Geometrien vorteilhaft sein. D.h. die Fig. 8 zeigt den Konnektor 1 mit der Drückfunktion in einer spanend gefertigten Ausführungsform.

Fig. 9 zeigt den Konnektor 1 gemäß einer vierten Ausführungsform. Der Konnektor 1 ist wie in Fig. 8 (aus einem Block) gefräst bzw. anderweitig spanend aus einem Stück gefertigt. Dabei ist der Konnektor der vierten Ausführungsform aber derart ausgestaltet, dass der Verschlussbügel 10 ziehbar ist. Der Vorsprung 10 mit der Einrastnut 28 sind also im Wesentlichen in einer Symmetrieachse der weiblichen Konnektorkomponente 4 angeordnet, die sich insbesondere in Längsrichtung der weiblichen Konnektorkomponente 4 erstreckt.

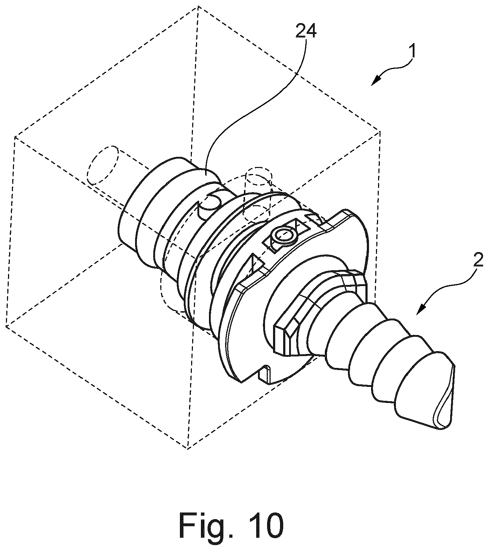

Fig. 10 zeigt eine weibliche Konnektorkomponente 4 gemäß einer weiteren Ausführungsform. Die beiden Konnektorkomponenten 2, 4 können entweder durch den Verschlussbügel 10 (hier nicht dargestellt) oder durch einen Gewindestift/Zylinderstift 34 (als gestrichelte Linie angedeutet) fest miteinander verbunden werden. Dabei kann ein Gewindestift/Zylinderstift 34 durch eine Aussparung der männlichen Konnektorkomponente 2 in die weibliche Konnektorkomponente 4 eingeschraubt/eingeschlagen werden. Somit kann die Konnektorverbindung zusätzlich gegen ein ungewolltes Lösen der Konnektorkomponenten 2, 4 gesichert werden. Die weibliche Konnektorkomponente 4 weist ferner die Dichtung 24 auf.

Der Verschlussbügel 10 kann Abschnitte mit unterschiedlichen (Innen-) Durchmessern aufweisen, insbesondere können die beiden frei auskragenden Schenkel des U-förmigen Bügels unterschiedlich Abstände über deren Schenkellänge definieren. D.h. der Benutzer kann beispielsweise auf einen Endabschnitt des Verschlussbügels (Querbalken des U-förmigen Bügels) 10 drücken, der gleichzeitig der Endabschnitt mit dem größeren Durchmesser ist. Durch den äußeren Druck wird der Endabschnitt in Richtung zu den beiden Konnektorkomponenten 2, 4 bzw. hin zum zentralen Fluidkanal geschoben, wodurch die frei auskragenden Schenkel schneller auseinandergedrückt werden und somit die männliche Konnektorkomponente 2 schneller freigeben. In einer anderen Ausführungsform kann der Benutzer den Verschlussbügel 10 aber auch nach außen ziehen, um die männliche Konnektorkomponente 2 freizugeben. In diesem Fall befindet sich der Endabschnitt mit dem größerenSchenkelabstand gegenüber von dem Abschnitt, an dem der Nutzer zieht.

Dabei ist der Verschlussbügel 10 derart ausgestaltet, dass ein seitlicher Endabschnitt des Verschlussbügels 10 einen größeren Durchmesser / Schenkelabstand aufweist als ein zentraler Abschnitt. Wenn der Verschlussbügel 10 derart verschoben wird, dass der Endabschnitt mit dem größeren Durchmesser / Schenkelabstand an der Rampe 14 anliegt, kann die Rampe 14 durch den Verschlussbügel 10 hindurchgeschoben werden. Somit ist der Einführabschnitt 6 freigegeben und kann relativ zu dem Aufnahmeabschnitt 8 verschoben werden. D. h. in dem Endabschnitt ist der Durchmesser / Schenkelabstand des Verschlussbügels 10 größer als der Außendurchmesser der vorstehenden Rampe 14. Nach dem Verschieben des Verschlussbügels 10 ist der Verschlussbügel 10 also nicht mehr im Eingriff in der Verrastnut 12. Der Verschlussbügel 10 kann derart vorgespannt sein, dass er nach dem Verschieben immer wieder in seine Ausgangsposition zurückkehrt. Dafür kann der Verschlussbügel 10 beispielsweise eine Vorspann-Feder aufweisen oder aus dieser bestehen.

Zusammengefasst betrifft die Offenbarung ein Fluidleitungs-Konnektorsystem mit einer männlichen und einer weiblichen Konnektorkomponente, die zur Herstellung einer Fluidverbindung zusammensteckbar sind, wofür die männliche Konnektorkomponente einen (dorn- oder steckerartigen) Einführabschnitt und die weibliche Konnektorkomponente einen, einen Aufnahmeraum ausbildenden Aufnahmeabschnitt (entsprechend einer Steckdose) hat, in welchen der Einführabschnitt einführ- /einsteckbar ist, und mit einem vorzugsweise bügelartigen Rastelement, das an/in der weiblichen Konnektorkomponente, vorzugsweise deren Aufnahmeabschnitt (quer zur Einsteckrichtung) verschiebbar gelagert ist und dabei (zumindest abschnittsweise) den Aufnahmeraum durchdringt/durchquert, derart, dass beim Einstecken des Einführabschnitts in den Aufnahmeraum das Rastelement durch den Einführabschnitt (zumindest teilweise) aus dem Aufnahmeraum nach Außen (selbsttätig) elastisch verdrängt/verbogen wird und mit Erreichen einer Fluidverbindungsendposition (vollständige Konnektion) in einen am Einführabschnitt angeordneten/ausgebildeten Hinterschnitt (Nut oder Vorsprung) verrastend zurückfedert (um ein Herausziehen des Einführabschnitts aus dem Aufnahmeraum zu blockieren), und dass beim (manuellen/nicht selbsttätigen) Verschieben des Rastelements quer zur Einsteckrichtung das Rastelement mittels einer an der weiblichen Konnektorkomponente ausgebildeten/angeordneten Rampe oder Abgleitfläche/-kante weg von dem Hinterschnitt, vorzugsweise aus dem Aufnahmeraum elastisch verdrängt/verbogen wird und so die Blockade für ein Herausziehen der Einführabschnitts aus dem Aufnahmeabschnitt aufhebt. Auf diese Weise wird die vergleichsweise leichtgängige (manuelle) Verschiebebewegung des Rastelements in eine vergleichsweise kraftaufwändige Verbiegebewegung des Rastelements für ein Freigeben der Konnektorverbindung transformiert und so die Bedienbarkeit verbessert.

### Bezugszeichenliste

- 1: Konnektor
- 2: männliche Konnektorkomponente
- 4: weibliche Konnektorkomponente
- 6: Einführabschnitt
- 8: Aufnahmeabschnitt
- 10: Verschlussbügel (Rastelement)
- 12: Nut
- 14: Rampe
- 16: Vorsprung
- 18: erster (Anschluss-)Stutzen
- 20: zweiter (Anschluss-)Stutzen
- 22: Fluidleitung
- 24: Dichtung
- 26: Dichtungsnut
- 28: Einrastnut
- 30: Hinterschnitt
- 32: Montageschräge
- 34: Gewindestift/Zylinderstift
- 36: Aufnahmeblech
- 38: Aufnahmenut
- 40: Schnapphaken

## Patentansprüche

1. Medizinischer Konnektor (1) zum lösbaren Herstellen einer Fluidverbindung zwischen einer männlichen Konnektorkomponente (2) des Konnektors (1) und einer weiblichen Konnektorkomponente (4) des Konnektors (1) durch Einführen eines Einführabschnitts (6) der männlichen Konnektorkomponente (2) in einen Aufnahmeabschnitt (8) der weiblichen Konnektorkomponente (4), wobei
ein Rastelement (10) der weiblichen Konnektorkomponente (4) beim Verbinden der beiden Konnektorkomponenten (2, 4) an einem entgegen der Einführrichtung wirkenden Hinterschnitt, vorzugsweise in eine Nut (12) an dem Einführabschnitt (6) einrastbar ist und
das Rastelement (10) zum Lösen der beiden Konnektorkomponenten (2, 4) quer, vorzugsweise senkrecht zu der Einführrichtung verschiebbar an der weiblichen Konnektorkomponente (4) gelagert ist,
**dadurch gekennzeichnet, dass**
das Rastelement (10) zumindest teilweise federelastisch ist, derart, dass es beim Verschieben in Richtung hin zu einer Mittelachse der beiden Konnektorkomponenten (2, 4) quer zur Einführrichtung nach radial innen durch einen Vorsprung (16) an der weiblichen Konnektorkomponente (4) für ein Lösen des Rasteingriffs mit dem Hinterschnitt deformierbar ist.

2. Konnektor (1) nach Anspruch 1, **dadurch gekennzeichnet, dass** das Rastelement (10) zum Lösen der beiden Konnektorkomponenten (2, 4) nach radial außen, weg von den beiden Konnektorkomponenten (2, 4) verschiebbar und, insbesondere ziehbar, ist.

3. Konnektor (1) nach Anspruch 2, **dadurch gekennzeichnet, dass** das Rastelement (10) beim Ziehen nach radial außen durch den Vorsprung (16) an der weiblichen Konnektorkomponente (4) deformierbar und insbesondere aufbiegbar ist.

4. Konnektor (1) nach einem der Ansprüche 1 bis 3, **dadurch gekennzeichnet, dass** der Vorsprung (16) eine Rampe an der weiblichen Konnektorkomponente (4) ist, an der ein Abschnitt des Rastelements (10) abgleitet, wobei das Rastelement (10) durch das Abgleiten entlang der Rampe aufbiegbar ist.

5. Konnektor (1) nach einem der Ansprüche 1 bis 4, **dadurch gekennzeichnet, dass** die weibliche Konnektorkomponente (4) in Verschiebungsrichtung des Rastelements (10) hinter dem Vorsprung (16) eine Einrast- oder Haltenut (28) aufweist, in die das Rastelement (10) nach dem Aufbiegen durch den Vorsprung (16) einrastet und so den Verschiebeweg begrenzt.

6. Konnektor (1) nach einem der Ansprüche 1 bis 5, **dadurch gekennzeichnet, dass** die Einrast- oder Haltenut (28) in einer Symmetrieachse der weiblichen Konnektorkomponente (4) angeordnet ist, die sich insbesondere in Längsrichtung der weiblichen Konnektorkomponente (4) erstreckt.

7. Konnektor (1) nach einem der Ansprüche 1 bis 6, **dadurch gekennzeichnet, dass** die männliche Konnektorkomponente (2) eine Rampe (14) aufweist, die nach radial außen aus dem Einführabschnitt (6) in den Aufnahmeabschnitt (8) vorsteht und das Rastelement (10) beim Einführen der männlichen Konnektorkomponente (2) durch die Rampe (14) elastisch verformbar, insbesondere aufbiegbar, ist.

8. Konnektor (1) nach einem der Ansprüche 1 bis 7, **dadurch gekennzeichnet, dass** das das Rastelement (10) ein U-förmiger Bügel ist, mit zwei frei auskragenden Schenkeln, deren Abstand zueinander bei einem Verschieben des Rastelements (10) quer zur Einführrichtung in eine Konnektor-Freigaberichtung durch den Vorsprung (6) elastisch vergrößert wird.

9. Konnektor (1) nach Anspruch 8, **gekennzeichnet durch** eine Montageschräge (32) zum Montieren des U-förmigen Rastelements (10) in eine Führung auf Seiten der weiblichen Konnektorkomponente (4).

10. Konnektor (1) nach einem der Ansprüche 1 bis 9, **dadurch gekennzeichnet, dass** der Konnektor (1), vorzugsweise die weibliche Konnektorkomponente (4), Schnapphaken (40) aufweist, durch die der Konnektor (1) in eine Aufnahmenut (38) eines Aufnahmeblechs (36) einrastbar ist, wobei insbesondere mehrere Konnektoren (1) neben- oder übereinander angeordnet sein können.
